(19) **Europäisches Patentamt / European Patent Office / Office européen des brevets**

(11) **EP 4 588 564 A1**

(12) # EUROPEAN PATENT APPLICATION
published in accordance with Art. 153(4) EPC

(43) Date of publication:
**23.07.2025 Bulletin 2025/30**

(21) Application number: **23865149.1**

(22) Date of filing: **09.08.2023**

(51) International Patent Classification (IPC):
*B01J 31/28* (2006.01)      *B01J 37/02* (2006.01)
*B01J 37/16* (2006.01)      *C07B 61/00* (2006.01)
*C07C 67/055* (2006.01)      *C07C 69/15* (2006.01)

(52) Cooperative Patent Classification (CPC):
**B01J 31/28; B01J 37/02; B01J 37/16; C07B 61/00;
C07C 67/055; C07C 69/15**

(86) International application number:
**PCT/JP2023/029103**

(87) International publication number:
**WO 2024/057790 (21.03.2024 Gazette 2024/12)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **16.09.2022  JP 2022148220**

(71) Applicant: **Resonac Corporation
Tokyo 105-7325 (JP)**

(72) Inventors:
- **KITAGAWA, Kazuhiro**
  **Tokyo 105-8518 (JP)**
- **KIMURA, Yojiro**
  **Tokyo 105-8518 (JP)**
- **UMEHARA, Kazuki**
  **Tokyo 105-8518 (JP)**
- **HOSOGI, Yasuhiro**
  **Tokyo 105-8518 (JP)**
- **IWAMA, Yasuhiro**
  **Tokyo 105-8518 (JP)**

(74) Representative: **Strehl & Partner mbB
Maximilianstrasse 54
80538 München (DE)**

(54) **METHOD FOR MANUFACTURING CATALYST FOR MANUFACTURE OF VINYL ACETATE AND METHOD FOR MANUFACTURING VINYL ACETATE**

(57)    A method for manufacturing a catalyst for manufacture of vinyl acetate, the catalyst containing a carrier, copper, palladium, gold, and an acetate, wherein the method comprises, in the following order: step 1) a step for impregnating the carrier with an alkaline solution; step 2) a step for contact-impregnating the carrier with a solution that contains a copper-containing compound, a palladium-containing compound, and a gold-containing compound, the amount of the solution exceeding a desired amount of carried catalyst components; step 3) a step for separating the carrier from the solution once the desired amount of carried catalyst components is carried by the carrier; step 4) a step for performing reduction treatment; and step 5) a step for causing the carrier to carry the acetate.

**Description**

Technical Field

The present disclosure relates to a method for producing a catalyst for vinyl acetate production, which is used when vinyl acetate is produced from acetic acid, ethylene, and oxygen as raw materials, and a method for producing vinyl acetate using the catalyst.

Background Art

Vinyl acetate is an important industrial material which is used as a raw material for a vinyl acetate resin, a raw material for polyvinyl alcohol, and a monomer for copolymerization with ethylene, styrene, acrylate, methacrylate or the like in a wide range of fields, such as paints, adhesives, and fiber treating agents.

As a catalyst for vinyl acetate production from acetic acid, ethylene and oxygen as raw materials, a catalyst in which palladium, gold and potassium acetate are supported on silica is widely used. An active site in this reaction is considered to be palladium, and gold is considered to have a role of suppressing aggregation of palladium and, additionally, reducing generation of a by-product carbon dioxide gas, thereby improving selectivity of vinyl acetate. The presence of gold atoms close to palladium is necessary for the effect of gold to be exhibited. In Patent Literature 1, palladium and gold are supported in a state in which supporting portions of palladium and gold are close to each other by devising a step of impregnation of a carrier.

In vinyl acetate production, it is an important technical problem to increase the selectivity of vinyl acetate, and suppression of generation of carbon dioxide gas is desired also from the viewpoint of environmental load.

In Patent Literature 2, the selectivity of vinyl acetate is improved by supporting copper in addition to palladium and gold.

Patent Literature 3 describes a method for producing a catalyst containing palladium, gold and copper, in which a palladium-containing compound, a gold-containing compound and a copper-containing compound are supported on a carrier in the same step, thereby improving the selectivity of vinyl acetate.

Citation List

Patent Literature

PTL 1: JP 2008-080326 A
PTL 2: JP 2002-516749 T
PTL 3: WO 2022/113429

Summary of Invention

Technical Problem

In Patent Literature 3, an alkali component is supported on a carrier, then the carrier is impregnated with a solution containing a palladium-containing compound, a gold-containing compound, and a copper-containing compound in the same step, and each compound is hydrolyzed, whereby a palladium hydroxide, a gold hydroxide, and a copper hydroxide are supported on the carrier. In general, a gold-containing compound represented by chloroauric acid is slowly hydrolyzed and supported on a carrier at a low rate. Therefore, even if a palladium-containing compound and a gold-containing compound are brought into contact with an alkali component-supporting carrier in the same step, gold atoms which are not close to palladium may be generated due to a difference in hydrolysis rate between palladium and gold, or the gold-containing compound remaining in the solution may be aggregated and coarsened to generate an aggregate of gold particles. However, when the copper-containing compound coexists during the hydrolysis of the gold-containing compound, the copper-containing compound is rapidly hydrolyzed and supported on the carrier, and then the gold-containing compound is adsorbed onto a hydrolysate of the copper-containing compound supported on the carrier, whereby the hydrolysis rate of the gold-containing compound can be increased. Therefore, as compared with a case where the copper-containing compound is not added in the same step, gold can be supported in a state of being close to palladium, and coarsening of gold particles in the solution can be suppressed, and, as a result, the selectivity of vinyl acetate can be improved. However, since aggregation of gold particles is also observed in the catalyst produced by this production method, further improvement in performance of the catalyst can be expected if gold aggregation in a hydrolysis step of catalyst production can be further suppressed.

The present disclosure provides a method for producing a catalyst capable of producing vinyl acetate with improved selectivity while ensuring high catalytic activity.

Solution to Problem

As a result of earnest studies for solving the above problems, the present inventors have found a method for producing a catalyst, characterized in that: when compounds containing copper, palladium, and gold, respectively, are supported on a carrier while the compounds are reacted with an alkali component, the compounds containing the respective metals are charged in excess relative to amounts of the compounds containing the metals corresponding to desired supported amounts of the metals; at a time point when the supported amounts of the metals reach the desired values, a solution of the compounds containing the metals is separated from the carrier; and a reduction treatment is performed. The present inventors have succeeded in vinyl acetate production with an improved selectivity.

The present disclosure includes the following aspects [1] to [9].

[1] A method for producing a catalyst for vinyl acetate production containing a carrier, copper, palladium, gold and an acetate, the method comprising:

Step 1: impregnating the carrier with an alkali solution;
Step 2: subjecting the carrier to contact impregnation with a solution containing a copper-containing compound, a palladium-containing compound, and a gold-containing compound in excessive amounts relative to desired supported amounts of catalyst components which are copper, palladium, and gold, respectively;
Step 3: separating the carrier from the solution when the catalyst components are supported on the carrier in the desired supported amounts of the catalyst components;
Step 4: performing a reduction treatment; and
Step 5: supporting an acetate on the carrier,
in this order.

[2] The production method according to any one of aspect [1], wherein, in Step 2, an excess rate of a charged amount of the gold-containing compound relative to a desired supported amount of gold is from 2% to 150%.
[3] The production method according to aspect [1] or [2], wherein, in Step 2, an excess rate of a charged amount of the palladium-containing compound relative to a desired supported amount of palladium is from 2% to 50%.
[4] The production method according to any one of aspects [1] to [3], wherein, in Step 2, an excess rate of a charged amount of the copper-containing compound relative to a desired supported amount of copper is from 2% to 50%.
[5] The production method according to any one of aspects [1] to [4], wherein a mass of supported metallic copper per 1 kg of the carrier is from 0.25 g to 5.00 g.
[6] The production method according to any one of aspects [1] to [5], wherein a mass of supported metallic palladium per 1 kg of the carrier is from 5.00 g to 20.0 g.
[7] The production method according to any one of aspects [1] to [6], wherein a mass of supported metallic gold per 1 kg of the carrier is from 4.00 g to 20.0 g.
[8] The production method according to any one of aspects [1] to [7], wherein a mass of supported acetate per 1 kg of the carrier is from 40 g to 125 g.
[9] A method for producing vinyl acetate, wherein a catalyst for vinyl acetate production obtained by the method according to any one of aspects [1] to [8] is used, and ethylene, oxygen and acetic acid are used as raw materials.

Advantageous Effects of Invention

According to the method of the present disclosure, it is possible to suppress aggregation of gold particles, and to produce a catalyst for vinyl acetate production having an alloy state of palladium and gold suitable for a reaction for generating vinyl acetate. As a result, the selectivity of vinyl acetate can be remarkably improved as compared with known methods while securing high catalytic activity.

Description of Embodiments

Embodiments of the present invention will be described below. However, it should be noted that the present invention is not limited to these embodiments, and various modifications can be made within the scope of the present invention.

In the present specification, when "to" is used for a numerical range, numerical values on both ends are an upper limit value and a lower limit value, respectively, and are included in the numerical range.

Method for Producing Catalyst for Vinyl Acetate Production

A method for producing a catalyst for vinyl acetate production according to an embodiment comprises the following steps in this order. Copper, palladium, and gold are sometimes referred to as "catalyst components", and a copper-containing compound, a palladium-containing compound, and a gold-containing compound are sometimes referred to as "raw material compounds" collectively or referred to as "raw material compound" for each metal.

Step 1: impregnating a carrier with an alkali solution;
Step 2: subjecting the carrier to contact impregnation with a solution (hereinafter also referred to as a "solution A") containing a copper-containing compound, a palladium-containing compound, and a gold-containing compound in excessive amounts relative to desired supported amounts of catalyst components which are copper, palladium, and gold, respectively;
Step 3: separating the carrier from the solution A when the catalyst components are supported on the carrier in the desired supported amounts thereof;
Step 4: performing a reduction treatment;
Step 5: supporting an acetate on the carrier.

In an embodiment, Step 2 is performed after Step 1, and the carrier is subjected to contact impregnation with the solution A containing a copper-containing compound, a palladium-containing compound, and a gold-containing compound to form a catalyst precursor in which reaction products of these compounds with an alkaline compound are supported on the carrier.

Steps 1 to 5 are performed in the above-described order, but other steps may be included for the purpose of improving the performance of the catalyst or the like. Examples of such steps include a step of bringing the carrier into contact with a pH buffer solution after Step 3 and a step of washing the carrier with water after Step 4. The reduction treatment of Step 4 is a treatment for converting the reaction products of the raw material compounds with the alkaline compound into metallic copper, metallic palladium, and metallic gold, respectively, and therefore must be performed after Step 3. Step 4 and Step 5 may be exchanged exceptionally. Hereinafter, each step will be described in detail.

Step 1. Impregnating Carrier with Alkali Solution

In this step, a carrier is impregnated with an alkali solution. This step can be carried out at room temperature. Upon completion of the impregnation operation, the carrier may be dried, or the process may proceed to the next step without any operation, such as drying.

The carrier is not particularly limited, and a porous substance generally used as a carrier for a catalyst can be used. The carrier is preferably silica, alumina, silica-alumina, diatomaceous earth, montmorillonite or titania, and more preferably silica. When a material mainly composed of silica is used as the carrier, a silica content of the carrier is usually at least 50 mass%, suitably at least 90 mass%, based on a mass of the carrier.

A specific surface area of the carrier as measured by a BET method is preferably at least 0.01 $m^2$/g, more preferably in a range of from 10 to 1000 $m^2$/g, and particularly preferably in a range of from 100 to 500 $m^2$/g. A bulk density of the carrier is preferably in a range of from 50 to 1000 g/L, and particularly preferably in a range of from 400 to 500 g/L. A water absorption rate of the carrier is preferably in a range of from 0.05 to 3 g-water/g-carrier, and particularly preferably in a range of from 0.1 to 2 g-water/g-carrier. With respect to a pore structure of the carrier, an average pore diameter is preferably in a range of from 1 to 1000 nm, and particularly preferably in a range of from 2 to 800 nm. When the average pore diameter is 1 nm or more, gas diffusion can be facilitated. On the other hand, when the average pore diameter is 1000 nm or less, the specific surface area of the carrier necessary for obtaining the catalytic activity can be secured.

A mercury intrusion method and a gas adsorption method (BJH method) are widely used to measure a pore diameter distribution of the carrier. In terms of classification of pores by IUPAC (International Union of Pure and Applied Chemistry), it is possible to measure macropores of 50 nm or more and some of mesopores of from 2 nm to less than 50 nm by the mercury intrusion method, and mesopores and micropores of 2 nm or less by the gas adsorption method. An appropriate measurement method can be selected according to the size of the pore diameter.

In the present disclosure, the water absorption rate of the carrier refers to a numerical value as measured by the following measurement method.

1. Weigh about 5 g of the carrier (W1 g) with a balance and place the carrier in a 100 mL beaker.
2. Add about 15 mL of pure water (ion-exchanged water) to the beaker to completely cover the carrier.
3. Allow the beaker to stand for 30 minutes.

4. Put the contents of the beaker on a wire mesh and remove the pure water.

5. Remove water attached to a surface of the carrier by lightly pressing it with a paper towel until the surface is no longer glossy.

6. Measure a total mass of the carrier and the pure water (W2 g).

7. Calculate the water absorption rate of the carrier from the following equation:

$$\text{Water absorption rate (g-water/g-carrier)} = (W2 - W1)/W1$$

Therefore, a water absorption amount (g) of the carrier is calculated by the water absorption rate (g-water/g-carrier) of the carrier $\times$ the mass (g) of the carrier used.

A shape of the carrier is not particularly limited. Specific examples include, but are not limited to, powders, spheres, and pellets. An optimum shape can be selected in accordance with a reaction mode, a reactor and the like to be used.

A particle size of the carrier is also not particularly limited. When the carrier is spherical, a particle diameter thereof is preferably in a range of from 1 to 10 mm, and more preferably in a range of from 3 to 8 mm. When the catalyst is packed in a tubular reactor and a gas phase reaction is carried out, and the particle diameter is 1 mm or more, an excessive increase in pressure loss during gas flow can be prevented and gas circulation can be effectively carried out. On the other hand, when the particle diameter is 10 mm or less, it is easy to diffuse a raw material gas to the inside of the catalyst, and a catalytic reaction can be effectively progressed. In addition, since the number of catalyst particles packed in the tubular reactor is not excessively reduced, it is possible to secure a total surface area of the catalyst particles sufficient to attain an amount of a metal component (copper, palladium, gold, or the like) which is dispersed on the surface of the carrier, appropriate for the reaction.

The alkali solution may be a solution of any alkaline compound. Examples of the alkaline compound include hydroxides of alkali metals or alkaline earth metals, bicarbonates of alkali metals or alkaline earth metals, carbonates of alkali metals or alkaline earth metals, and silicates of alkali metals or alkaline earth metals. As the alkali metal, lithium, sodium or potassium can be used. Barium or strontium can be used as the alkaline earth metal. As the alkaline compound, sodium metasilicate, potassium metasilicate, sodium hydroxide, potassium hydroxide, barium hydroxide or strontium hydroxide is suitably used.

A solvent of the alkali solution is not particularly limited, and examples thereof include water, methanol, and ethanol. The solvent is preferably water.

The alkaline compound is used in excess relative to a total of desired supported amounts of copper, palladium and gold, which will be described later. For example, a product of a molar amount of the alkaline compound and a valence of the alkaline compound is preferably more than 1.1 times and 3.0 times or less, and more preferably more than 1.5 times and 2.0 times or less of a sum of a product of a molar amount of the palladium-containing compound and a valence of palladium, a product of a molar amount of the gold-containing compound and a valence of gold, and a product of a molar amount of the copper-containing compound and a valence of copper.

A method for impregnating the carrier with the alkali solution is not particularly limited. Examples of the method include (I) a method in which a carrier is immersed in a large amount of an alkali solution for a while and then the carrier impregnated with the alkali solution in an amount corresponding to a water absorption amount is taken out, and (II) a method in which an alkaline compound is dissolved in a solvent and a carrier is impregnated with a solution diluted in a measuring flask to an amount correspond to a water absorption amount of the carrier. From the viewpoint of a waste liquid treatment, the method (II) is desirable.

The carrier is preferably impregnated with the alkali solution in an amount corresponding to from 0.9 times by mass to 1.0 times by mass the water absorption amount of the carrier, and more preferably impregnated with the alkali solution in an amount corresponding to from 0.95 times by mass to 1.0 times by mass the water absorption amount of the carrier. When the amount of the alkali solution is 0.9 times by mass or more the water absorption amount of the carrier, uneven impregnation of the alkali solution is less likely to occur. When the amount of the alkali solution is 1.0 times by mass or less the water absorption amount of the carrier, the entire amount of the alkali solution can be reliably absorbed by the carrier. In the present disclosure, the water absorption amount of the carrier is a value as measured with pure water, is strictly different from a value with respect to the alkali solution, but is used as it is for convenience.

Step 2. Subjecting Carrier to Contact Impregnation with Solution A

In this step, the carrier impregnated with the alkali solution is subjected to contact impregnation with the solution A. The solution A is a solution containing a copper-containing compound, a palladium-containing compound, and a gold-containing compound. The solution A may contain other components as necessary.

In the solution A, raw material compounds (a copper-containing compound, a palladium-containing compound,

and a gold-containing compound) are charged in excessive amounts obtained by back calculation from supported amounts of catalyst components (metallic copper, metallic palladium, and metallic gold) in a desired catalyst composition, i.e., a finally obtained catalyst. That is, the raw material compounds are charged in amounts obtained by multiplying the amounts of the raw material compounds corresponding to the desired supported amounts of the catalyst components by excess rates so that all of the copper-containing compound, the palladium-containing compound, and the gold-containing compound are in excess relative to the desired supported amounts of the catalyst components. Concentrations of the raw material compounds of the catalyst components (copper, palladium, and gold) in the solution A can be calculated from the amounts of the raw material compounds calculated as described above and the solution amount. In actual operation, required amounts (g) of the raw material compounds may be weighed and dissolved, and a preferable solution amount may be attained.

The excess rate of the charged amount of the raw material compound of each catalyst component in the solution A is calculated by the following equation:

(Amount (mass) of catalyst component in charged raw material compound - amount (mass) of desired catalyst component to be supported)/(amount (mass) of desired catalyst component to be supported) × 100 (%).

When the catalyst component is gold, the amount (g) of the gold-containing compound (e.g., chloroauric acid) corresponding to the amount (g) of gold to be supported per 1 kg of the carrier is calculated, and the gold-containing compound is dissolved in the solvent of the solution A, in an amount (g) obtained by multiplying this calculated amount by the amount (kg) of the carrier and (excess rate of gold + 100)/100. Concretely, when the amount (g) of gold to be supported per 1 kg of the carrier is 10 g, the amount of chloroauric acid ($HAuCl_4$) containing 10 g of gold atoms is 17.26 g. When the excess rate is 80%, the charged amount of the chloroauric acid per 1 kg of the carrier is 31.068 g (= 17.26 × ((80 + 100)/100). The chloroauric acid is dissolved in the solvent of solution A, in an amount obtained by multiplying this value by the amount (kg) of the carrier to be used. For palladium and copper, the solution A is prepared in the same manner.

The excess rate of the charged amount of the gold-containing compound in the solution A is preferably 2% or more, more preferably 10% or more, and still more preferably 20% or more. The excess rate of the charged amount of the gold-containing compound is preferably 150% or less, more preferably 100% or less, and still more preferably 90% or less. These upper limit values and lower limit values can be arbitrarily combined. The excess rate of the charged amount of the gold-containing compound in the solution A is preferably from 2% to 150%, more preferably from 10% to 100%, and still more preferably from 20% to 90%. The excess rate of the charged amount of the palladium-containing compound in the solution A is preferably 2% or more, more preferably 4% or more, and still more preferably 10% or more. The excess rate of the charged amount of the palladium-containing compound is preferably 50% or less, more preferably 40% or less, and still more preferably 20% or less. These upper limit values and lower limit values can be arbitrarily combined. The excess rate of the charged amount of the palladium-containing compound in the solution A is preferably from 2% to 50%, more preferably from 4% to 40%, and still more preferably from 10% to 20%. The excess rate of the charged amount of the copper-containing compound in the solution A is preferably 2% or more, more preferably 2.5% or more, and still more preferably 8% or more. The excess rate of the charged amount of the copper-containing compound is preferably 50% or less, more preferably 40% or less, and still more preferably 15% or less. These upper limit values and lower limit values can be arbitrarily combined. The excess rate of the charged amount of the copper-containing compound in the solution A is preferably from 2% to 50%, more preferably from 2.5% to 40%, and still more preferably from 8% to 15%.

The use of the solution A containing excessive amounts of the raw material compounds relative to the desired supported amounts of the catalyst components can shorten a contact time between the carrier and the solution A required for obtaining the desired supported amounts of the catalyst components. As a result, coarsening of gold particles due to dissolution and reprecipitation processes is suppressed.

As the copper-containing compound in the solution A, a copper precursor which can be converted into metallic copper can be used. Examples of the copper precursor which can be converted into metallic copper include copper chloride, copper acetate and copper nitrate, and copper chloride is preferably used.

As the palladium-containing compound in the solution A, a palladium precursor which can be converted into metallic palladium can be used. Examples of the palladium precursor which can be converted into metallic palladium include palladium chloride, palladium nitrate, palladium sulfate, sodium chloropalladate, potassium chloropalladate, barium chloropalladate, and palladium acetate, and sodium chloropalladate is preferably used.

As the gold-containing compound in the solution A, a gold precursor which can be converted into metallic gold can be used. Examples of the gold precursor include chloroauric acid, sodium chloroaurate, and potassium chloroaurate, and chloroauric acid is preferably used.

Examples of the solvent of the solution A include water, an alcohol, and an organic acid. Water is preferable, because it does not damage the carrier and has no reactivity with the compounds contained in the solution A.

An amount of the solution A is preferably from 1.0 to 10.0 times by mass, more preferably from 2.0 to 8.0 times by mass, and particularly preferably from 2.0 to 5.0 times by mass the water absorption amount of the carrier.

Upon contact of the carrier impregnated with the alkali solution with the solution A, the metal compounds as the raw materials can be converted into water-insoluble substances, and a shell-shaped catalyst precursor can be formed in which the metal components such as palladium, gold, and copper are unevenly present and supported on a surface portion of the carrier.

A contact temperature is not particularly limited, but is preferably from 10 to 80°C, and more preferably from 20 to 60°C. When the contact temperature is 10°C or higher, a conversion reaction can be sufficiently progressed. When the contact temperature is 80°C or lower, aggregation of copper, palladium, and gold can be suppressed.

The contact time is set to a time at which the desired supported amounts of the catalyst components are attained, after confirmation of a temporal change in supported rates of the catalyst components at a predetermined contact temperature through repeated preliminary experiments. Since the contact time cannot be changed for each catalyst component, the excess rate of the charged amount of each raw material compound is adjusted, so that the desired supported amount thereof is obtained during the contact time. The contact time is preferably from 0.5 to 100 hours, and more preferably from 1 to 20 hours. By setting the contact time to 100 hours or less, deterioration of the carrier can be suppressed.

Step 3. Separating Carrier from Solution

In this step, only the carrier is taken out from the mixture of the carrier and the solution obtained in Step 2. Since the gold-containing compound, such as chloroauric acid has a significantly lower hydrolysis rate than those of the palladium-containing compound and the copper-containing compound, long-term immersion of the carrier in the solution A is likely to cause unevenness in composition in the carrier. In addition, when the carrier is immersed in the solution A for a long time, coarsening of gold particles is likely to occur in the dissolution and reprecipitation processes. On the other hand, the occurrence of unevenness in composition and the coarsening of gold particles can be suppressed by separating the carrier from the solution A when the desired amounts of the catalyst components are supported on the carrier. A method for separating the carrier and the solution from each other is not particularly limited, and can be appropriately determined by a known method in consideration of the shape, form and the like of the catalyst. For example, a filtration method, a centrifugal separation method, a sedimentation method or the like can be used, and a filtration method is preferable. Examples of the filtration method include a method of passing through a mesh smaller than the size of the carrier. When the carrier size is from 3 to 8 mm, the carrier may be passed through a mesh having an opening size of 2 mm, for example. The solution is preferably separated, by solid-liquid separation, up to 1.2 times or less the water absorption rate of the carrier.

Step 4. Performing Reduction Treatment

Desirably, the carrier supporting reaction products of the raw material compounds and the alkaline compound is subjected to a reduction treatment to convert the reaction products into metallic palladium, metallic gold, and metallic copper. In the present disclosure, the "metallic copper", the "metallic palladium", and the "metallic gold" refer to metal species having a valence of 0. The reduction treatment can be carried out by either liquid phase reduction or gas phase reduction. Not all palladium, gold, and copper may be reduced to the metallic states, i.e., a valence of 0, by the reduction treatment.

The liquid phase reduction can be carried out either in a non-aqueous system using an alcohol or a hydrocarbon or in an aqueous system. As a reducing agent, for example, a carboxylic acid and a salt thereof, an aldehyde, hydrogen peroxide, a saccharide, a polyhydric phenol, a boron compound, an amine, or hydrazine can be used. Examples of the carboxylic acid and salt thereof include oxalic acid, potassium oxalate, formic acid, potassium formate, potassium citrate, and ammonium citrate. Examples of the aldehyde include formaldehyde and acetaldehyde. Examples of the saccharide include glucose. Examples of the polyhydric phenol include hydroquinone. Examples of the boron compound include diborane and sodium borohydride. As the reducing agent, hydrazine, formaldehyde, acetaldehyde, hydroquinone, sodium borohydride and potassium citrate are preferable, and hydrazine is particularly preferable.

When the liquid phase reduction is performed, a temperature therefor is not particularly limited, but a liquid phase temperature is preferably in a range of from -10 to 100°C, and more preferably in a range of from 0 to 50°C. When the liquid phase temperature is -10°C or higher, a sufficient reduction rate can be obtained. On the other hand, when the liquid phase temperature is 100°C or lower, aggregation of copper, palladium, and gold can be suppressed. A reduction time is not particularly limited, but is preferably in a range of from 0.5 to 24 hours, and more preferably in a range of from 1 to 10 hours. When the reduction time is 0.5 hours or more, the reduction can be sufficiently progressed. On the other hand, when the reduction time is 24 hours or less, aggregation of copper, palladium, and gold can be suppressed.

The reducing agent used in the gas phase reduction can be selected from, for example, hydrogen gas, carbon monoxide, alcohols, aldehydes, and olefins, such as ethylene, propene, and isobutene. The reducing agent is preferably hydrogen gas. In the gas phase reduction, an inert gas may be added as a diluent. Examples of the inert gas include helium, argon, and nitrogen gas.

When the gas phase reduction is performed, a temperature therefor is not particularly limited, but the impregnated carrier is preferably heated to a range of from 30 to 350°C, and more preferably to a range of from 100 to 300°C. When the heating temperature is 30°C or higher, a sufficient reduction rate can be obtained. On the other hand, when the heating temperature is 300°C or lower, aggregation of copper, palladium, and gold can be suppressed. A reduction time is not particularly limited, but is preferably in a range of from 0.5 to 24 hours, and more preferably in a range of from 1.0 to 10 hours. When the reduction time is 0.5 hours or more, the reduction can be sufficiently progressed. On the other hand, when the reduction time is 24 hours or less, aggregation of copper, palladium, and gold can be suppressed.

A pressure for the gas phase reduction is not particularly limited, but is preferably in a range of from 0.0 to 3.0 MPaG (gauge pressure), and more preferably in a range of from 0.1 to 1.0 MPaG (gauge pressure) from the viewpoint of equipment.

A feed rate of the reducing agent in the case of performing the gas phase reduction is preferably in a range of from 10 to 15000 $hr^{-1}$, and particularly preferably in a range of from 100 to 8000 $hr^{-1}$ in terms of space velocity (hereinafter referred to as SV) in a standard state.

The carrier subjected to the reduction treatment is washed with pure water or the like as necessary. Washing may be carried out continuously or batchwise. A washing temperature is preferably in a range of from 5 to 200°C, and more preferably in a range of from 15 to 80°C. A washing time is not particularly limited, and conditions sufficient for the purpose of removing the remaining undesirable impurities may be selected. Undesirable impurities include, for example, chlorine-containing compounds and chloride ions. After washing, the carrier may be dried by heating, if necessary.

Step 5. Supporting Acetate on Carrier

An acetate can be supported by, for example, impregnating the carrier with a solution containing a required amount of the acetate and drying the impregnated carrier. An amount of the acetate solution to be used is preferably from 0.9 to 1.0 times by mass the water absorption amount of the carrier. The acetate is supported typically after the reduction treatment, but may also be supported before the reduction treatment.

The acetate is preferably at least one compound selected from the group consisting of an alkali metal acetate and an alkaline earth metal acetate, and more preferably an alkali metal acetate. Examples of the alkali metal acetate include acetates of lithium, sodium, and potassium. As the acetate, sodium acetate and potassium acetate are preferable, and potassium acetate is particularly preferable.

Catalyst for Vinyl Acetate Production

A mass of the supported metallic copper per 1 kg of the carrier is preferably 0.25 g or more, more preferably 0.60 g or more, and still more preferably 0.80 g or more. The mass of the supported metallic copper per 1 kg of the carrier is preferably 5.00 g or less, more preferably 4.50 g or less, and still more preferably 4.00 g or less. These upper limit values and lower limit values can be arbitrarily combined. The mass of the supported metallic copper per 1 kg of the carrier is preferably from 0.25 g to 5.00 g, more preferably from 0.60 g to 4.50 g, and still more preferably from 0.80 g to 4.00 g.

A mass of the supported metallic palladium per 1 kg of the carrier is preferably 5.00 g or more, more preferably 8.00 g or more, and still more preferably 10.0 g or more. The mass of the supported metallic palladium per 1 kg of the carrier is preferably 20.0 g or less, more preferably 19.0 g or less, and still more preferably 17.0 g or less. These upper limit values and lower limit values can be arbitrarily combined. The mass of the supported metallic palladium per 1 kg of the carrier is preferably from 5.00 g to 20.0 g, more preferably from 8.00 g to 19.0 g, and still more preferably from 10.0 g to 17.0 g.

A mass of the supported metallic gold per 1 kg of the carrier is preferably 4.00 g or more, more preferably 6.00 g or more, and still more preferably 8.00 g or more. The mass of the supported metallic gold per 1 kg of the carrier is preferably 20.0 g or less, more preferably 18.0 g or less, and still more preferably 17.0 g or less. These upper limit values and lower limit values can be arbitrarily combined. The mass of the supported metallic gold per carrier 1 kg is preferably from 4.00 g to 20.0 g, more preferably from 6.00 g to 18.0 g, and still more preferably from 8.00 g to 17.0 g.

A mass of the supported acetate per 1 kg of the carrier is preferably 40 g or more, and more preferably 50 g or more. The mass of the supported acetate per 1 kg of the carrier is preferably 125 g or less, and more preferably 88 g or less. These upper limit values and lower limit values can be arbitrarily combined. The mass of the supported acetate per 1 kg of the carrier is preferably from 40 g to 125 g, and more preferably 50 g to 88 g.

According to one embodiment, the catalyst for vinyl acetate production obtained by the method of the present disclosure has an eggshell structure in which most of copper, palladium, and gold are supported on the surface portion of the carrier. A thickness of a shell portion varies depending on the kinds of the carrier, the alkali solution, and the solution containing the raw material metal compounds to be used. When spherical silica having a diameter of 5 mm is used as the carrier, the shell portion has a thickness of preferably from 0.05 to 0.5 mm, and more preferably from 0.1 to 0.3 mm. When the shell portion has a thickness of 0.05 mm or more, the catalytic activity can be maintained even when the surface portion of the carrier is peeled off during the reaction. When the shell portion has a thickness of 0.5 mm or less, the catalyst concentration at the surface portion of the carrier can be sufficiently increased, and merits of shell-shaped supporting can be economically enjoyed. The acetate does not need to be supported in a shell shape and may be present uniformly throughout the catalyst.

Specific Example of Catalyst Production

A specific example of catalyst production will be described below.

1. A carrier is impregnated with an alkali solution in an amount corresponding to a water absorption amount of the carrier.
2. The carrier is immersed, for a predetermined time, in a solution A obtained by diluting, in a measuring flask, raw material metal compounds of copper, palladium and gold in excessive amounts relative to desired supported amounts of catalyst components, with pure water up to an amount of 2 times by mass the water absorption amount of the carrier, and the carrier is thus subjected to contact impregnation with the solution A to form a catalyst precursor on which the desired amounts of the catalyst components are supported.
3. The catalyst precursor subjected to contact impregnation is separated from the solution obtained in 2.
4. The catalyst precursor obtained in 3 is brought into contact with a reducing agent to perform a reduction treatment.
5. The reduced carrier is washed with pure water.
6. The washed carrier is dried.
7. A predetermined amount of an acetate is supported on the carrier.
8. The carrier on which the catalyst components are supported is dried.

Vinyl Acetate Production

Hereinafter, a method for producing vinyl acetate using the catalyst for vinyl acetate production produced by the method of the present disclosure will be described. The reaction for producing vinyl acetate is preferably carried out in a gas phase using acetic acid (HOAc), ethylene, and oxygen as reaction raw materials. The gas phase reaction may be in any form known in the art, but is preferably a fixed bed flow reaction.

A reaction formula is as follows:

$$CH_2=CH_2 + CH_3COOH + 1/2O_2 \rightarrow CH_2=CHOCOCH_3 + H_2O$$

A ratio among acetic acid, ethylene, and oxygen in a raw material gas is preferably acetic acid: ethylene: oxygen = 1: from 0.08 to 16: from 0.01 to 4, and more preferably acetic acid: ethylene: oxygen = 1: from 0.2 to 9: from 0.07 to 2 as a molar ratio.

The raw material gas contains ethylene, acetic acid (gas), and oxygen gas, and may further contain nitrogen gas, carbon dioxide, a rare gas, or the like as a diluent if necessary. When ethylene, acetic acid, and oxygen are defined as reaction raw materials, a ratio of the reaction raw materials to the diluent is preferably reaction raw materials: diluent = 1: from 0.05 to 9, and more preferably reaction raw materials: diluent = 1: from 0.1 to 3 as a molar ratio.

When the reaction is carried out by a fixed bed flow reaction, the raw material gas is fed to a reactor at a space velocity (SV) of preferably from 10 to 15000 hr$^{-1}$, and more preferably from 300 to 8000 hr$^{-1}$ in a standard state. By setting the space velocity to 10 hr$^{-1}$ or more, heat of reaction can be appropriately removed. On the other hand, when the space velocity is 15000 hr$^{-1}$ or less, the size of equipment, such as a compressor can be a practical size.

Into the raw material gas, water is preferably added, as water vapor, in an amount of from 0.5 to 20 mol%, and more preferably from 1 to 18 mol%. Without wishing to be bound by any theory, it is believed that the presence of water in the reaction system inhibits outflow of the acetate from the catalyst. Even if water is added in an amount of more than 20 mol%, not only the above effect is not improved, but also the hydrolysis of vinyl acetate may proceed. Therefore, the presence of a large amount of water in the raw material gas is not preferable.

A material for the reactor is not particularly limited, but is preferably a material having corrosion resistance.

A reaction temperature is preferably in a range of from 100 to 300°C, and more preferably in a range of from 120 to 250°C. When the reaction temperature is 100°C or higher, an appropriate reaction speed can be maintained. When the reaction temperature is 300°C or lower, heat of reaction can be easily removed.

A reaction pressure is preferably in a range of from 0 to 3 MPaG (gauge pressure), and more preferably in a range of from 0.1 to 1.5 MPaG. If the reaction pressure is 0 MPaG or more, an appropriate reaction speed can be maintained. If the reaction pressure is 3 MPaG or less, it is not necessary to increase the pressure resistance of equipment, such as a reaction tube, and an equipment cost can be reduced.

The reaction raw material ethylene to be used is preferably high-purity ethylene, but may have a lower saturated hydrocarbon, such as methane, ethane or propane mixed therein.

The oxygen gas is also not particularly limited. An oxygen gas diluted with an inert gas, such as nitrogen gas or carbon dioxide gas (for example, in the form of air) can be fed. However, when the reaction gas is circulated, it is generally advantageous to use a high-concentration oxygen gas, suitably an oxygen gas having a purity of 99% or more.

Examples

The present invention will be described in more detail below by way of examples, but is not limited to these examples alone.

Example 1: Preparation of Catalyst A

A catalyst was prepared using a silica spherical carrier (particle diameter: 5 mm, specific surface area: 155 m$^2$/g, water absorption rate: 0.85 g-water/g-carrier) according to the following procedure.

Step 1. A carrier (23.5 g) (water absorption amount: 20.0 g) was impregnated with an aqueous solution containing $Na_2SiO_3 \cdot 9H_2O$: 5.3 g in an amount corresponding to the water absorption amount of the carrier (1.1 times by mass). The container containing the carrier and the aqueous solution was shaken to completely impregnate the carrier with the solution, and the impregnated carrier was allowed to stand in the closed container for 3 hours. The water absorption amount was calculated from the carrier amount of 23.5 g and the water absorption rate of 0.85 g-water/g-carrier (the same applies to the following examples and comparative examples).

Step 2. The carrier obtained in Step 1 was immersed in an aqueous solution containing $Na_2PdCl_4$: 1.0 g, $HAuCl_4$: 0.80 g, and copper chloride dihydrate: 0.23 g in an amount of 2 times by mass the water absorption amount of the carrier, and allowed to stand at room temperature (20°C) for 2 hours to obtain a catalyst precursor on which desired amounts of catalyst components were supported. The relationship between the amounts and time until the catalyst components were supported was obtained through a preliminary experiment, and the standing time was determined from the time at which desired supported amounts were attained. The desired supported amounts of the catalyst components were 13.4 g/kg for palladium, 11.1 g/kg for gold and 3.3 g/kg for copper per 1 kg of the carrier, and the excess rates of the charged amounts of the respective raw material compounds relative to the desired supported amounts were set to 16.5% for the palladium-containing compound, 77.9% for the gold-containing compound, and 10.2% for the copper-containing compound.

Step 3: The catalyst precursor was separated from the solution obtained in Step 2 using a metallic mesh having an opening of 1 mm, followed by liquid removal.

Additional step: The catalyst precursor obtained in Step 3 was immersed at 20°C in a pH buffer solution (pH = 10.0) containing $NaHCO_3$: 0.86 g and $Na_2CO_3$: 0.68 g in an amount of 4 times by mass the water absorption amount of the carrier, and allowed to stand at room temperature (20°C) for 18 hours. Then, the catalyst precursor was separated from the pH buffer solution using the same metallic mesh as that described above, followed by liquid removal.

Step 4: The catalyst precursor obtained in the additional step was immersed in an aqueous solution containing 6.1 g of a 52 mass% aqueous hydrazine hydrate solution in an amount of 2 times by mass the water absorption amount of the carrier, and allowed to stand at room temperature (20°C) for 4 hours to perform a reduction treatment. The palladium/gold/copper/carrier composition was then washed with deionized water, and water washing was continued until the water after washing was free of chloride ions. The washed palladium/gold/copper/carrier composition was dried at 110°C for 4 hours.

Step 5: The palladium/gold/copper/carrier composition obtained in Step 4 was impregnated at room temperature (20°C) with an aqueous solution containing 1.7 g of potassium acetate in an amount corresponding to 0.9 times by mass the water absorption amount of the carrier, allowed to stand for 0.5 hours, and hot-air dried at 110°C for 4 hours to obtain a catalyst A.

Example 2: Preparation of Catalyst B

The same operation as in Example 1 was performed to obtain a catalyst B, except that the desired supported amounts of the catalyst components were changed to 13.5 g/kg for palladium, 10.8 g/kg for gold, and 3.3 g/kg for copper per 1 kg of the carrier, that the excess rates of the charged amounts of the raw material compounds relative to the desired supported amounts were changed as shown in Table 1, and that the additional step was not carried out.

Comparative Example 1: Preparation of Catalyst C

A catalyst C was prepared using a silica spherical carrier (particle diameter: 5 mm, specific surface area: 155 m$^2$/g, water absorption rate: 0.85 g-water/g-carrier) according to the following procedure in accordance with Example 1 of Patent Literature 3 (WO 2022/113429).

Step [1]. A carrier (23.5 g) (water absorption amount: 20.0 g) was impregnated with an aqueous solution containing $Na_2SiO_3 \cdot 9H_2O$: 2.3 g in an amount corresponding to the water absorption amount of the carrier (0.95 times by mass). The container containing the carrier and the aqueous solution was shaken to completely impregnate the carrier with the solution, and the impregnated carrier was air dried for 5 minutes.

Step [2]. The carrier obtained in Step [1] was immersed in an aqueous solution containing $Na_2PdCl_4$: 0.92 g, $HAuCl_4$: 0.28 g, and copper chloride dihydrate: 0.04 g in an amount of 2 times by mass the water absorption amount of the carrier, and allowed to stand at room temperature for 20 hours to obtain a catalyst precursor.

Step [3]. To the mixture of the catalyst precursor and the aqueous solution obtained in Step [2], 1.9 g of a 52 mass% aqueous hydrazine hydrate solution was added, and the mixture was gently mixed, and allowed to stand at room temperature (20°C) for 4 hours to perform a reduction treatment. The resulting palladium/gold/copper/carrier composition was then washed with deionized water, and water washing was continued until the water after washing was free of chloride ions. The washed palladium/gold/copper/carrier composition was hot-air dried at 110°C for 4 hours.

Step [4]. The palladium/gold/copper/carrier composition was impregnated at room temperature (20°C) with an aqueous solution containing 1.7 g of potassium acetate in an amount corresponding to 0.9 times by mass the water absorption amount of the carrier, and hot-air dried at 110°C for 4 hours to obtain a catalyst C. The excess rates of the charged amounts of the respective raw material compounds relative to the supported amounts of the metals of the catalyst C determined by measurement of the supported amounts of the metals (copper, palladium, and gold) and potassium acetate which will be described below were 5.1% for the palladium-containing compound, 8.2% for the gold-containing compound, and 0% of the copper-containing compound.

Comparative Example 2: Preparation of Catalyst D

A catalyst D was prepared using a silica spherical carrier (particle diameter: 5 mm, specific surface area: 155 $m^2/g$, water absorption rate: 0.85 g-water/g-carrier) according to the following procedure in accordance with Examples of Patent Literature 2 (JP 2002-516749 T).

Step [1]. A carrier (23.5 g) (water absorption amount: 20.0 g) was impregnated with an aqueous solution containing $Na_2PdCl_4$: 0.88 g and copper chloride dihydrate: 0.13 g in an amount corresponding to the water absorption amount of the carrier (0.95 times by mass). The container containing the carrier and the aqueous solution was shaken to completely impregnate the carrier with the solution.

Step [2]. The carrier obtained in Step [1] was brought into contact with a mixed solution containing 0.7 g of a 50 mass% aqueous NaOH solution and 35.7 g of distilled water for 2.5 hours to immobilize palladium and copper on the carrier as palladium (II) and copper hydroxide.

Step [3]. The palladium (II)/copper hydroxide/carrier composition obtained in Step [2] was washed with deionized water, and water washing was continued until the water after washing was free of chloride ions. After water washing, the palladium (II)/copper hydroxide/carrier composition was dried at a temperature of 150°C for 10 hours under a nitrogen gas flow. Then, the composition was brought into contact with ethylene (5 mol% in nitrogen gas) in a gas phase at 150°C for 5 hours to perform a reduction treatment. The resulting palladium/copper/carrier composition was allowed to cool over 10 hours, washed with deionized water for 2 hours, and dried in an oven at 150°C for 5 hours.

Step [4]. Gold hydroxide (0.2 g) was mixed with 0.10 g of potassium hydroxide in 17 mL of water, the resulting orange suspension was heated to 85°C, and all solids were dissolved over 5 hours to obtain a clear yellow solution of potassium aurate. The yellow solution was added to the palladium/copper/carrier composition obtained in Step [3], and the carrier was impregnated with the solution over 30 minutes. Thereafter, the composition was dried for 5 hours in an oven at 100°C under a nitrogen gas flow. Then, a reduction treatment was performed with ethylene (5 mol% in nitrogen gas) at 120°C for 5 hours to obtain free metallic gold on the carrier.

Step [5]. The palladium/gold/copper/carrier composition obtained in Step [4] was impregnated with an aqueous solution containing 1.7 g of potassium acetate in an amount corresponding to 0.9 times by mass the water absorption amount of the carrier, and dried at 110°C for 4 hours to obtain a catalyst D. The excess rates of the charged amounts of the respective raw material compounds relative to the supported amounts of the metals of the catalyst D determined by measurement of the supported amounts of the metals (copper, palladium, and gold) and potassium acetate which will be described below were 0% for the palladium-containing compound, 0% for the gold-containing compound, and 0% for the copper-containing compound.

Evaluation of Catalyst

Measurement of Supported Amounts of Metals (Copper, Palladium, and Gold) and Potassium Acetate

A sample (3 g) of the supported catalyst was crushed and pressed into a disk having an internal diameter of 3 cm. The amount of metal in the disk was measured with a fluorescent X-ray spectroscopic analyzer ZSX PrimusII available from Rigaku Corporation. As for potassium acetate, the amount of potassium atoms was determined and converted into the amount of potassium acetate.

Catalytic activity evaluation test

The catalyst (6.7 mL) was diluted with 75 mL of glass bead and packed into a reaction tube (made of SUS316L, internal diameter: 22 mm, length: 480 mm). A gas having a composition of $C_2H_4/O_2/H_2O/HOAc/N_2 = 45/6/5/23/21$ (mol%) was allowed to flow at a flow rate of 66.7 NL/h for 240 hours under conditions: a reaction temperature of 150°C and a reaction pressure of 0.6 MPaG to stabilize the catalyst state. The vinyl acetate activity at 72 hours from the start of the reaction is shown in Table 1. Thereafter, the flow rate of the gas was changed to 20.0 NL/h and, further, the reaction temperature was changed to 145°C, 150°C, 155°C, or 160°C to evaluate the catalytic activity and selectivity. When the gas flow rate and the reaction temperature were changed, the catalytic activity and selectivity were evaluated after at least 4 hours at which the states of the apparatus and the catalyst stabilized. Generally, the higher the vinyl acetate activity (STY) (g/L-cat·h), the lower the vinyl acetate selectivity. Therefore, the vinyl acetate selectivities at the same vinyl acetate activity for the respective catalysts were compared. For example, the vinyl acetate selectivity at a vinyl acetate activity of 800 (g/L-cat·h) was calculated by interpolation from a polynomial approximation curve formed by plotting the vinyl acetate activity and the vinyl acetate selectivity at each reaction temperature.

The reactor outlet gas was analyzed using the following method.

1. Oxygen

Using an absolute calibration curve method, 50 mL of an effluent gas was collected, and the total amount of the effluent gas was allowed to flow into a 1 mL gas sampler attached to gas chromatography, followed by analysis under the following conditions.

Gas chromatography: gas chromatography (GC-14B available from Shimadzu Corporation) equipped with a gas sampler for Shimadzu gas chromatograph (MGS-4; measuring tube: 1 mL) Column: MS-5A IS60/80 mesh (3 mm $\Phi \times$ 3 m)

Carrier gas: helium (flow rate: 20 mL/min)
Temperature conditions: The detector temperature and vaporization chamber temperature were 110°C, and the column temperature was constant at 70°C.

Detector: TCD (He pressure: 70 kPaG, Current 100 mA)

2. Acetic acid

Using an internal standard method, 0.2 $\mu$L of an analytical solution prepared by adding 1 mL of 1,4-dioxane as an internal standard to 10 mL of the reaction solution was injected and analyzed under the following conditions.

Gas chromatography: GC-14B available from Shimadzu Corporation
Column: packed column Thermon 3000 (length: 3 m, internal diameter: 0.3 mm)
Carrier gas: nitrogen (flow rate: 20 mL/min)
Temperature conditions: The detector temperature and vaporization chamber temperature were 180°C, the column temperature was held at 50°C for 6 minutes from the start of analysis, then raised to 150°C at a rate of temperature rise of 10°C/min, and held at 150°C for 10 minutes. Detector: FID ($H_2$ pressure: 40 kPaG, air pressure: 100 kPaG)

3. Vinyl acetate

Using the internal standard method, 0.3 $\mu$L of an analytical solution prepared by adding 1 g of 1,4-dioxane as an internal standard to 6 g of the reaction solution was injected and analyzed under the following conditions.

Gas chromatography: GC-9A available from Shimadzu Corporation
Column: capillary column TC-WAX (length: 30 m, internal diameter: 0.25 mm, membrane thickness: 0.5 $\mu$m)
Carrier gas: nitrogen (flow rate: 30 mL/min)
Temperature conditions: The detector temperature and vaporization chamber temperature were 200°C, the column temperature was held at 45°C for 2 minutes from the start of analysis, then raised to 130°C at a rate of temperature rise of 4°C/min, held at 130°C for 15 minutes, then raised to 200°C at a rate of temperature rise of 25°C/min, and held at 200°C for 10 minutes. Detector: FID ($H_2$ pressure: 60 kPaG, air pressure: 100 kPaG)

The evaluation results of the catalysts are shown in Table 1. The selectivity of vinyl acetate is based on ethylene.

Table 1

| | Catalyst | Step 2 | | | Additional step | | Supported amount (g/kg) | | | | Catalyst performance evaluation | |
|---|---|---|---|---|---|---|---|---|---|---|---|---|
| | | Charged amount excess rate (%) | | | pH of aqueous solution | Type of aqueous solution | Per 1 kg of carrier | | | | Vinyl acetate selectivity (%) 1) | Vinyl acetate activity (g/L-cat·h) 2) |
| | | Pd | Au | Cu | | | Pd | Au | Potassium | Cu acetate | | |
| Example 1 | Catalyst A | 16.5 | 77.9 | 10.2 | 10.0 | NaHCO3/Na2CO3 | 13.4 | 11.1 | 3.3 | 70.2 | 95.4 | 720.0 |
| Example 2 | Catalyst B | 4.7 | 23.1 | 2.8 | - | - | 13.5 | 10.8 | 3.3 | 70.2 | 94.7 | 642.0 |
| Comparative Example 1 | Catalyst C | 5.1 | 8.2 | 0 | - | - | 13.5 | 6.4 | 0.6 | 70.2 | 94.2 | 946.0 |
| Comparative Example 2 | Catalyst D | 0 | 0 | 0 | - | - | 13.5 | 6.6 | 2.1 | 70.2 | 90.5 | 358.0 |

1) Values at a vinyl acetate activity (STY) [g/L-cat·h] of 800 for the catalysts A to C A value at a reaction temperature of 150°C for the catalyst D because of a significantly low vinyl acetate activity
2) A value at 72 hours from the start of the reaction

EP 4 588 564 A1

A comparison between Example 2 and Comparative Example 1 verifies that an improvement in vinyl acetate selectivity by 0.5 points and a particularly remarkable effect are obtained by the following procedure: when compounds containing palladium, gold, and copper, respectively, are supported on a carrier while the compounds are reacted with an alkali component, the respective metal raw material compounds are charged in excessive amounts relative to target supported amounts of catalyst components; and at a time point when the supported amounts of the catalyst components reach the target values, an aqueous solution of the metal raw material compounds is separated from the carrier.

Industrial Applicability

The present disclosure makes it possible to provide a catalyst for vinyl acetate production having excellent selectivity while securing high catalytic activity. The catalyst for vinyl acetate production is industrially useful.

**Claims**

1. A method for producing a catalyst for vinyl acetate production containing a carrier, copper, palladium, gold and an acetate, the method comprising:

   Step 1: impregnating the carrier with an alkali solution;
   Step 2: subjecting the carrier to contact impregnation with a solution containing a copper-containing compound, a palladium-containing compound, and a gold-containing compound in excessive amounts relative to desired supported amounts of catalyst components which are copper, palladium, and gold, respectively;
   Step 3: separating the carrier from the solution when the catalyst components are supported on the carrier in the desired supported amounts of the catalyst components;
   Step 4: performing a reduction treatment; and
   Step 5: supporting an acetate on the carrier,
   in this order.

2. The production method according to any one of claim 1, wherein, in Step 2, an excess rate of a charged amount of the gold-containing compound relative to a desired supported amount of gold is from 2% to 150%.

3. The production method according to claim 1 or 2, wherein, in Step 2, an excess rate of a charged amount of the palladium-containing compound relative to a desired supported amount of palladium is from 2% to 50%.

4. The production method according to claim 1 or 2, wherein, in Step 2, an excess rate of a charged amount of the copper-containing compound relative to a desired supported amount of copper is from 2% to 50%.

5. The production method according to claim 1 or 2, wherein a mass of supported metallic copper per 1 kg of the carrier is from 0.25 g to 5.00 g.

6. The production method according to claim 1 or 2, wherein a mass of supported metallic palladium per 1 kg of the carrier is from 5.00 g to 20.0 g.

7. The production method according to claim 1 or 2, wherein a mass of supported metallic gold per 1 kg of the carrier is from 4.00 g to 20.0 g.

8. The production method according to claim 1 or 2, wherein a mass of supported acetate per 1 kg of the carrier is from 40 g to 125 g.

9. A method for producing vinyl acetate, wherein a catalyst for vinyl acetate production obtained by the method according to claim 1 or 2 is used, and ethylene, oxygen and acetic acid are used as raw materials.

## INTERNATIONAL SEARCH REPORT

| International application No. |
| --- |
| **PCT/JP2023/029103** |

| A. | CLASSIFICATION OF SUBJECT MATTER |
| --- | --- |

***B01J 31/28***(2006.01)i; ***B01J 37/02***(2006.01)i; ***B01J 37/16***(2006.01)i; ***C07B 61/00***(2006.01)i; ***C07C 67/055***(2006.01)i; ***C07C 69/15***(2006.01)i

FI:  B01J31/28 Z; B01J37/02 101A; B01J37/02 101D; B01J37/16; C07C69/15; C07C67/055; C07B61/00 300

According to International Patent Classification (IPC) or to both national classification and IPC

| B. | FIELDS SEARCHED |
| --- | --- |

Minimum documentation searched (classification system followed by classification symbols)

B01J21/00-38/74; C07B61/00; C07C1/00-409/44

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

| C. | DOCUMENTS CONSIDERED TO BE RELEVANT | |
| --- | --- | --- |

| Category* | Citation of document, with indication, where appropriate, of the relevant passages | Relevant to claim No. |
| --- | --- | --- |
| A | WO 2022/113429 A1 (SHOWA DENKO K.K.) 02 June 2022 (2022-06-02)<br>claims, paragraphs [0029]-[0038], examples | 1-9 |
| A | JP 2008-80326 A (SHOWA DENKO K.K.) 10 April 2008 (2008-04-10)<br>claims 1-8, examples | 1-9 |
| A | CN 103418444 A (CHINA PETROLEUM & CHEMICAL CORPORATION) 04 December 2013 (2013-12-04)<br>claims 10-14, 20, 21, paragraph [0066] | 1-9 |

| ☐ Further documents are listed in the continuation of Box C. | ☑ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **11 October 2023** | **24 October 2023** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2023/029103**

| Patent document cited in search report | | | Publication date (day/month/year) | Patent family member(s) | | | Publication date (day/month/year) |
|---|---|---|---|---|---|---|---|
| WO | 2022/113429 | A1 | 02 June 2022 | TW | 202220752 | A | |
| JP | 2008-80326 | A | 10 April 2008 | US | 2010/0168465 | A1 | |
| | | | | claims 1-8, examples | | | |
| | | | | EP | 2059340 | A1 | |
| | | | | CN | 101511473 | A | |
| | | | | KR | 10-2009-0025352 | A | |
| | | | | WO | 2008/029597 | A1 | |
| CN | 103418444 | A | 04 December 2013 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008080326 A
- JP 2002516749 T

- WO 2022113429 A